# FASCICULE DE BREVET EUROPEEN

(11) **EP 1 435 226 B1**
(45) Date de publication et mention de la délivrance du brevet: **05.04.2006**
(21) Numéro de dépôt: 03293129.7
(22) Date de dépôt: 12.12.2003
(51) Int. Cl.: A61K 8/81, A61Q 5/06, A61K 8/04, A61K 8/33

(54) **Dispositif aérosol comprenant une composition capillaire de coiffage dans un milieu aqueux, à base d' un copolymère de N-vinyl pyrrolidone et N-vinyl imidazole**
Aerosol-Sprühvorrichtung enthaltend ein wässriges haarfestigendes Mittel mit einem Copolymer auf Basis von N-Vinylpyrrolidon und N-Vinylimidazol
Aerosol spraying device containing an aqueous hairstyling composition comprising a copolymer of N-vinyl pyrrolidone and N-vinyl imidazole

(30) Priorité: 02.01.2003 FR 0300003
(43) Date de publication de la demande: 07.07.2004
(73) Titulaire: L'OREAL, 75008 Paris (FR)
(72) Inventeur: Dupuis, Christine, 75018 Paris (FR); Gawtrey, Jonathan, 92100 Boulogne (FR)
(74) Mandataire: Bourdeau, Françoise

(56) Documents cités:
- EP-A- 1 059 349
- WO-A-00/30594
- DE-A- 19 731 907
- FR-A- 2 715 841
- FR-A- 2 771 925

## Description

La présente invention concerne un dispositif aérosol comprenant une composition capillaire de coiffage dans un milieu aqueux propulsée par le diméthyléther, à base d'un copolymère de N-vinyl pyrrolidone et N-vinyl imidazole, une utilisation dudit dispositif et un procédé de traitement mettant en oeuvre ledit dispositif.

Les compositions capillaires pour la mise en forme et/ou le maintien de la coiffure les plus répandues sur le marché de la cosmétique sont des compositions à pulvériser essentiellement constituées d'une solution le plus souvent alcoolique et d'un ou plusieurs matériaux, généralement des résines polymères, dont la fonction est de former des soudures entre les cheveux, appelés matériaux fixants, en mélange avec divers adjuvants cosmétiques. Cette solution est généralement conditionnée soit dans un récipient aérosol approprié mis sous pression à l'aide d'un agent propulseur, soit dans un flacon pompe.

On connaît de nombreux systèmes aérosols destinés à fixer les chevelures, ces systèmes contenant d'une part une phase liquide (ou jus) et d'autre part un agent propulseur. Ce dernier a pour fonction d'assurer une pression permettant la pulvérisation de la phase liquide, et son application sur les cheveux sous forme d'un nuage de gouttelettes dispersées. Dans le souci de diminuer la quantité de composés organiques volatils (COV) tels que les alcools et les hydrocarbures fluorés ou Fréons présents dans ce type de composition à pulvériser et de respecter l'environnement, on s'intéresse plus particulièrement aux formulations aqueuses propulsées par le diméthyléther.

La phase liquide contient les matériaux fixants et un solvant approprié. C'est une fois appliquée sur les cheveux que la phase liquide sèche, permettant la formation de soudures nécessaires à la fixation de la chevelure par les matériaux fixants. Les soudures doivent être suffisamment rigides pour assurer le maintien des cheveux. Cependant, elles doivent également être suffisamment fragiles pour que l'utilisateur puisse, en peignant ou brossant la chevelure, les détruire sans heurter le cuir chevelu ni endommager les cheveux.

Des dispositifs aérosols contenant des compositions à base de diméthyléther et de polymère contenant des motifs N-vinyl lactame sont déjà connus et sont décrits par exemple dans la demande de brevet FR 2 771 925.

Néanmoins, il existe un réel besoin d'améliorer la fixation et le maintien des cheveux, c'est-à-dire d'obtenir des soudures de meilleures qualité, tout en gardant de bonnes propriétés cosmétiques, telles que la douceur ou le démêlage.

La demanderesse a maintenant découvert que l'utilisation d'au moins un copolymère à base de motifs de N-vinylpyrrolidone et de N-vinylimidazole particulier dans des compositions capillaires aqueuses particulières permet une amélioration de la fixation des cheveux tout en maintenant de bonnes propriétés cosmétiques.

La présente invention a donc pour objet un dispositif aérosol comprenant une composition capillaire aqueuse fixante à base d'au moins un copolymère à base de motifs de N-vinyl pyrrolidone et N-vinyl imidazole, et un agent propulseur comprenant le diméthyléther.

Un autre objet de l'invention consiste en l'utilisation dudit dispositif pour appliquer une laque sur les cheveux.

L'invention a également pour objet un produit à deux composants comprenant les compositions précitées.

L'invention a encore pour objet un procédé de traitement capillaire mettant en oeuvre ledit dispositif.

D'autres objets, caractéristiques, aspects et avantages de l'invention apparaîtront encore plus clairement à la lecture de la description et des divers exemples qui suivent.

La présente invention concerne un dispositif aérosol contenant une composition capillaire de coiffage qui comprend dans un milieu aqueux cosmétiquement acceptable au moins un copolymère à base de motifs de N-vinyl pyrrolidone et de N-vinyl imidazole ayant un poids moléculaire (ou PM) supérieur à 10 KDaltons, et du diméthyléther comme agent propulseur.

Le poids moléculaire des copolymères de l'invention est compris de préférence entre 100 et 5000 Kdaltons, et encore plus préférentiellement entre 500 et 1500 Kdaltons.

De préférence, le copolymère à base de motifs de N-vinyl pyrrolidone et de N-vinyl imidazole présente un module de Young supérieur à 600MPa, mieux encore compris entre 600 et 10000MPa. Ce module de Young ou module d'élasticité est une constante définie par le rapport entre la contrainte et la déformation pour un matériau donné. Il est mesuré à 25°C sur un film de 500 µm d'épaisseur et d'un taux d'humidité relative de 48% via l'appareillage LYOD L151.

Les copolymères préférés sont formés par 10 à 90% en poids du motif N-vinyl pyrrolidone et par 10 à 90% en poids du motif N-vinyl imidazole, et encore plus préférentiellement par 40 à 60% en poids du motif N-vinyl pyrrolidone et par 40 à 60% du motif N-vinyl imidazole.

De préférence, le copolymère utilisé est celui commercialisé sous la dénomination LUVITEC VPI 55 K61.

La composition capillaire de coiffage selon l'invention contient de préférence, de 0,1 à 15% en poids de copolymère par rapport au poids total de la composition.

Selon l'invention, la composition contient de préférence, en outre au moins un polymère fixant autre que les copolymères fixants de l'invention. Ces polymères fixants permettent de donner ou de maintenir une forme à la chevelure.

Les polymères fixants convenant dans l'invention sont ceux généralement utilisés dans la technique. Ils sont notamment choisis parmi les polymères anioniques, cationiques, amphotères, non ioniques et leurs mélanges.

Les polymères fixants peuvent être solubles dans le milieu cosmétiquement acceptable ou insolubles dans ce même milieu et utilisés dans ce cas sous forme de dispersions de particules solides ou liquides de polymère (latex ou pseudolatex).

Les polymères fixants anioniques généralement utilisés sont des polymères comportant des groupements dérivés d'acide carboxylique, sulfonique ou phosphorique et ont une masse moléculaire moyenne en nombre comprise entre environ 500 et 5 000 000.

Les groupements carboxyliques sont apportés par des monomères mono ou diacides carboxyliques insaturés tels que ceux répondant à la formule : 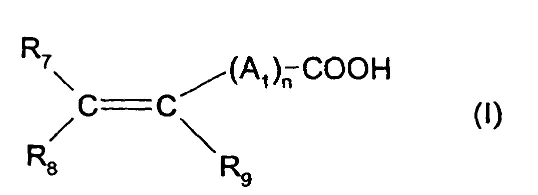 dans laquelle n est un nombre entier de 0 à 10, A₁ désigne un groupement méthylène, éventuellement relié à l'atome de carbone du groupement insaturé ou au groupement méthylène voisin lorsque n est supérieur à 1, par l'intermédiaire d'un hétéroatome tel qu'oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle.

Dans la formule précitée, un groupement alkyle inférieur désigne de préférence un groupement ayant 1 à 4 atomes de carbone et en particulier, les groupements méthyle et éthyle.

Les polymères fixants anioniques à groupements carboxyliques préférés selon l'invention sont :
A) Les homo- ou copolymères d'acide acrylique ou méthacrylique ou leurs sels et en particulier les produits vendus sous les dénominations VERSICOL® E ou K par la société ALLIED COLLOID et ULTRAHOLD® par la société BASF, les copolymères d'acide acrylique et d'acrylamide, les sels de sodium des acides polyhydroxycarboxyliques.
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkyléne-glycol tel que le polyéthylène-glycol, et éventuellement réticulés. De tels polymères sont décrits en particulier dans le brevet français n° 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois n^{os} 75370 et 75371. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀, par exemple, de lauryle, tels que celui commercialisé par la société ISP sous la dénomination ACRYLIDONE® LM et les terpolymères acide méthacrylique/acrylate d'éthyle/acrylate de tertiobutyle tels que le produit commercialisé sous la dénomination LUVIMER® 100 P par la société BASF.
C) Les copolymères dérivés d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés et réticulés, ou encore un autre monomère ester vinylique, allylique ou méthallylique d'un acide carboxylique α- ou β-cyclique. De tels polymères sont décrits entre autres dans les brevets français n^{os} 1 222 944, 1 580 545, 2 265 782, 2 265 781, 1 564 110 et 2 439 798. Un produit commercial entrant dans cette classe est la résine 28-29-30 commercialisée par la société National Starch.
D) Les copolymères dérivés d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US n^{os} 2 047 398, 2 723 248, 2 102 113, le brevet GB n° 839 805. Des produits commerciaux sont notamment ceux vendus sous les dénominations GANTREZ® AN
   ou ES par la société ISP.
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne, les fonctions anhydrides de ces copolymères étant éventuellement monoestérifiées ou monoamidifiées.

Ces polymères sont par exemple décrits dans les brevets français n^{os} 2 350 384 et 2 357 241 de la demanderesse.

E) Les polyacrylamides comportant des groupements carboxylates.

Les polymères comprenant les groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone.
- les sels de l'acide polystyrène-sulfonique tels que les sels de sodium vendus par exemple sous la dénomination Flexan® 130 par National Starch. Ces composés sont décrits dans le brevet FR 2 198 719.
- les sels d'acides polyacrylamide-sulfoniques tels que ceux mentionnés dans le brevet US 4 128 631, et plus particulièrement l'acide polyacrylamidoéthylpropane-sulfonique.

Selon l'invention, parmi les polymères fixants anioniques cités ci-dessus, on préférera les copolymères d'acide acrylique tels que les terpolymères acide acrylique/acrylate d'éthyle/N-tertiobutylacrylamide vendus notamment sous la dénomination ULTRAHOLD® STRONG par la société BASF, les copolymères dérivés d'acide crotonique tels que les terpolymères acétate de vinyle/tertio-butylbenzoate de vinyle/acide crotonique et les terpolymères acide crotonique/acétate de vinyle/ néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié vendus, par exemple, sous la dénomination GANTREZ® par la société ISP, les copolymères d'acide méthacrylique et de méthacrylate de méthyle vendus sous la dénomination EUDRAGIT® L par la société ROHM PHARMA, les copolymères d'acide méthacrylique et d'acrylate d'éthyle vendus sous la dénomination LUVIMER® MAEX ou MAE par la société BASF et les copolymères acétate de vinyle/acide crotonique et les copolymères acétate de vinyle/acide crotonique greffés par du polyéthylèneglycol vendus sous la dénomination ARISTOFLEX® A par la société BASF.

Les polymères fixants anioniques cités ci-dessus, les plus particulièrement préférés sont choisis parmi les copolymères méthylvinyléther/anhydride maléique monoestérifiés vendus sous la dénomination GANTREZ® ES 425 par la société ISP, les terpolymères acide acrylique/acrylate d'éthyle/N-tertiobutylacrylamide vendus sous la dénomination ULTRAHOLD® STRONG par la société BASF, les copolymères d'acide méthacrylique et de méthacrylate de méthyle vendus sous la dénomination EUDRAGIT® L par la société ROHM PHARMA, les terpolymères acétate de vinyle/tertio-butylbenzoate de vinyle/acide crotonique et les terpolymères acide crotonique/acétate de vinyle/ néododécanoate de vinyle vendus sous la dénomination Résine 28-29-30 par la société NATIONAL STARCH, les copolymères d'acide méthacrylique et d'acrylate d'éthyle vendus sous la dénomination LUVIMER® MAEX OU MAE par la société BASF, les terpolymères vinylpyrrolidone/acide acrylique/méthacrylate de lauryle vendus sous la dénomination ACRYLIDONE® LM par la société ISP.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques, ou bien B et C peuvent désigner des groupements dérivant de monomères zwittérioniques de carboxybétaïnes ou de sulfobétaïnes;

B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-α,β-dicarboxylique dont l'un des groupements carboxyliques a été amené à réagir avec une polyamine comportant un ou plusieurs groupements amine primaire ou secondaire.

Les polymères fixants amphotères répondant à la définition donnée ci-dessus plus particulièrement préférés sont choisis parmi les polymères suivants :
(1) les polymères résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique, tel que plus particulièrement les dialkylaminoalkylméthacrylate et acrylate, les dialkylaminoalkylméthacrylamide et acrylamide. De tels composés sont décrits dans le brevet américain n° 3 836 537.
(2) les polymères comportant des motifs dérivant :
   a) d'au moins un monomère choisi parmi les acrylamides ou les méthacrylamides substitués sur l'atome d'azote par un groupe alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou diéthyle.
      Les acrylamides ou méthacrylamides N-substitués plus particulièrement préférés selon l'invention sont les composés dont les groupes alkyle comportent de 2 à 12 atomes de carbone, et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.

   Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone des acides ou des anhydrides maléïque ou fumarique.
   Les comonomères basiques préférés sont des méthacrylates d'aminoéthyle, de butylaminoéthyle, de N,N'-diméthylaminoéthyle, de N-tertio-butylaminoéthyle.
   On utilise particulièrement les copolymères dont la dénomination CTFA (4ème Ed., 1991) est Octylacrylamide/acrylates/butylaminoethyl-methacrylate copolymer, tels que les produits vendus sous la dénomination AMPHOMER® ou LOVOCRYL® 47 par la société NATIONAL STARCH.
(3) les polyaminoamides réticulés et acylés partiellement ou totalement dérivant de poyaminoamides de formule générale :

   ⁅CO-R₁₀-CO-Z⁆ (II)

   dans laquelle R₁₀ représente un groupe divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un groupe dérivant de l'addition de l'un quelconque desdits acides avec une amine bis-primaire ou bis-secondaire, et Z désigne un groupe dérivant d'une polyalkylène-polyamine bis-primaire, mono- ou bis-secondaire et de préférence représente :
   a) dans les proportions de 60 à 100 % en moles, le groupe 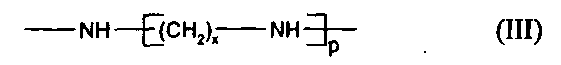 où x=2 et p=2 ou 3, ou bien x=3 et p=2
      ce groupe dérivant de la diéthylène-triamine, de la triéthylène-tétraamine ou de la dipropylène-triamine;
   b) dans les proportions de 0 à 40 % en moles, le groupe (IIIbis) ci-dessus, dans lequel x=2 et p=1 et qui dérive de l'éthylène-diamine, ou le groupe dérivant de la pipérazine : 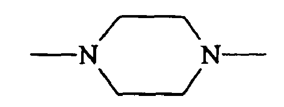
   c) dans les proportions de 0 à 20 % en moles, le groupe -NH-(CH₂)₆-NH- dérivant de l'hexaméthylènediamine, ces polyaminoamides étant réticulés par réaction d'addition d'un agent réticulant bifonctionnel choisi parmi les épihalohydrines, les diépoxydes, les dianhydrides, les dérivés bis-insaturés, au moyen de 0,025 à 0,35 mole d'agent réticulant par groupement amine du polyaminoamide, et acylés par action d'acide acrylique, d'acide chloracétique ou d'une alcane-sultone ou de leurs sels.
      Les acides carboxyliques saturés sont choisis de préférence parmi les acides ayant 6 à 10 atomes de carbone tels que les acides adipique, triméthyl-2,2,4-adipique et triméthyl-2,4,4-adipique, téréphtalique, les acides à double liaison éthylénique comme, par exemple, les acides acrylique, méthacrylique, itaconique.
      Les alcane-sultones utilisées dans l'acylation sont de préférence la propane- ou la butane-sultone, les sels des agents d'acylation sont de préférence les sels de sodium ou de potassium.
(4) les polymères comportant des motifs zwittérioniques formule : 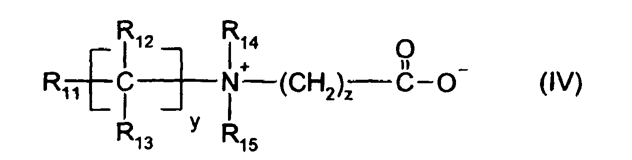 dans laquelle R₁₁ désigne un groupement insaturé polymérisable tel qu'un groupement acrylate, méthacrylate, acrylamide ou méthacrylamide, y et z représentent un nombre entier de 1 à 3, R₁₂ et R₁₃ représentent un atome d'hydrogène, un groupe méthyle, éthyle ou propyle, R₁₄ et R₁₅ représentent un atome d'hydrogène ou un groupe alkyle de telle façon que la somme des atomes de carbone dans R₁₄ et R₁₅ ne dépasse pas 10.
   Les polymères comprenant de telles unités peuvent également comporter des motifs dérivés de monomères non zwittérioniques tels que l'acrylate ou le méthacrylate de diméthyl- ou diéthylaminoéthyle ou des acrylates ou méthacrylates d'alkyle, des acrylamides ou méthacrylamides, ou l'acétate de vinyle.
   A titre d'exemple, on peut citer les copolymères méthacrylate de méthyle/diméthyl-carboxyméthylammonio-éthylméthacrylate de méthyle.
(5) les polymères dérivés du chitosane comportant des motifs monomères répondant aux formules suivantes : 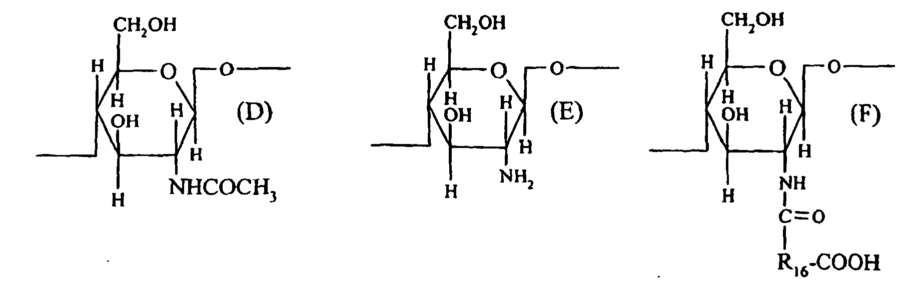 le motif (D) étant présent dans des proportions comprises entre 0 et 30%, le motif (E) dans des proportions comprises entre 5 et 50% et le motif (F) dans des proportions comprises entre 30 et 90%, étant entendu que dans ce motif (F), R₁₆ représente un groupe de formule : 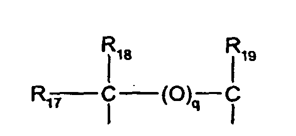 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes R₁₇, R₁₈ et R₁₉ étant dans ce cas un atome d'hydrogène ;
   ou si q=1, R₁₇, R₁₈ et R₁₉ représentent chacun un atome d'hydrogène, ainsi que les sels formés par ces composés avec des bases ou des acides.
(6) Les polymères répondant à la formule générale (V) sont, par exemple, décrits dans le brevet français 1 400 366 : 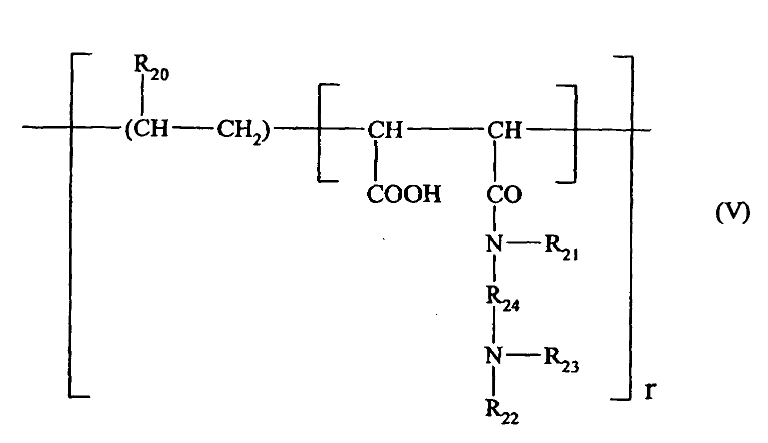 dans laquelle R₂₀ représente un atome d'hydrogène, un groupe CH₃O, CH₃CH₂O, phényle, R₂₁ désigne un atome d'hydrogène ou un groupe alkyle inférieur tel que méthyle, éthyle, R₂₂ désigne un atome d'hydrogène ou un groupe alkyle inférieur en C₁-C₆ tel que méthyle, éthyle, R₂₃ désigne un groupe alkyle inférieur en C₁-C₆ tel que méthyle, éthyle ou un groupe répondant à la formule : -R₂₄-N(R₂₂)₂, R₂₄ repré-sentant un groupement -CH₂-CH₂- , -CH₂-CH₂-CH₂-, -CH₂₋CH(CH₃)-, R₂₂ ayant les significations mentionnées ci-dessus.
(7) Les polymères dérivés de la N-carboxyalkylation du chitosane comme le N-carboxyméthyl-chitosane ou le N-carboxybutylchitosane.
(8) Les polymères amphotères du type -D-X-D-X choisis parmi:
   a) les polymères obtenus par action de l'acide chloracétique ou le chloracétate de sodium sur les composés comportant au moins un motif de formule :

      -D-X-D-X-D- (VI)

      où D désigne un groupe 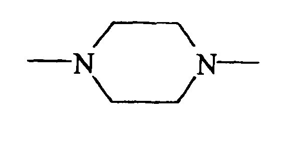 et X désigne le symbole E ou E', E ou E' identiques ou différents désignent un groupe bivalent qui est un groupe alkylène à chaîne droite ou ramifiée, comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule :

      -D-X-D-X- (VI')

      où D désigne un groupe 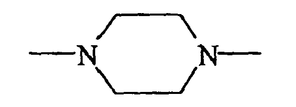 et X désigne le symbole E ou E' et au moins une fois E' ; E ayant la signification indiquée ci-dessus et E' est un groupe bivalent qui est un groupe alkylène à chaîne droite ou ramifiée, ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) les copolymères alkyl(C₁-C₅)vinyléther/anhydride maléique modifiés partiellement par semiamidification avec une N,N-dialkylaminoalkylamine telle que la N,N-diméthylaminopropylamine
ou par semiestérification avec un N,N-dialkylaminoalcanol. Ces copolymères peuvent également comporter d'autres comonomères vinyliques tels que le vinylcaprolactame.

Parmi les polymères fixants amphotères cités ci-dessus les plus particulièrement préférés selon l'invention, on citera ceux de la famille (3) tels que les copolymères dont la dénomination CTFA est Octylacrylamide/acrylates/butylamino-ethylmethacrylate copolymer, tels que les produits vendus sous les dénominations AMPHOMER®, AMPHOMER® LV 71 ou LOVOCRYL® 47 par la société NATIONAL STARCH et ceux de la famille (4) tels que les copolymères méthacrylate de méthyle/diméthyl-carboxyméthylammonio-éthylméthacrylate de méthyle.

Les polymères fixants non ioniques utilisables selon la présente invention sont choisis, par exemple, parmi :
- les polyalkyloxazolines ;
- les homopolymères d'acétate de vinyle ;
- les copolymères d'acétate de vinyle et d'ester acrylique ;
- les copolymères d'acétate de vinyle et d'éthylène ;
- les copolymères d'acétate de vinyle et d'ester maléïque, par exemple, de maléate de dibutyle ;
- les copolymères d'esters acryliques tels que, par exemple, les copolymères d'acrylates d'alkyle et de méthacrylates d'alkyle tels que les produits proposés par la société ROHM & HAAS sous les dénominations PRIMAL® AC-261 K et EUDRAGIT® NE 30 D, par la société BASF sous la dénomination 8845, par la société HOECHST sous la dénomination APPRETAN® N9212 ;
- les copolymères d'acrylonitrile et d'un monomère non ionique choisis, par exemple, parmi le butadiène et les (méth)acrylates d'alkyle ; on peut citer les produits proposés sous la dénomination CJ 0601 B par la société ROHM & HAAS ;
- les homopolymères de styrène ;
- les copolymères de styrène et de (méth)acrylate d'alkyle tels que les produits MOWILITH® LDM 6911, MOWILITH® DM 611 et MOWILITH® LDM 6070 proposés par la société HOECHST, les produits RHODOPAS® SD 215 et RHODOPAS® DS 910 proposés par la société RHONE POULENC ;
- les copolymères de styrène, de méthacrylate d'alkyle et d'acrylate d'alkyle ;
- les copolymères de styrène et de butadiène ;
- les copolymères de styrène, de butadiène et de vinylpyridine ;
- les copolymères d'acrylate d'alkyle et d'uréthanne ;
- les polyamides,
- les homopolymères et copolymères de vinyllactame.

Les groupes alkyle des polymères non ioniques mentionnés ci-dessus ont, de préférence, de 1 à 6 atomes de carbone.

Selon la présente invention, les polymères fixants non-ioniques à motifs vinyllactames peuvent être ceux décrits dans les brevets US 3 770 683, US 3 929 735, US 4 521 504, US 5 158 762, US 5 506 315 et dans les demandes de brevet WO 94/121148, WO 96/06592 et WO 96/10593. Ils peuvent se présenter sous forme pulvérulente ou sous forme de solution ou de suspension.

Les homopolymères ou copolymères à motifs vinyllactame comprennent des motifs de formule (IX): 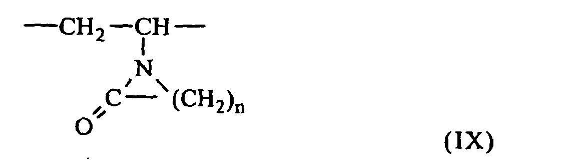 dans laquelle n est indépendamment 3, 4 ou 5.

La masse moléculaire en nombre des polymères à motifs vinyllactames est généralement supérieure à environ 5 000, de préférence comprise entre 10 000 et 1 000 000 environ, plus préférentiellement comprise entre 10 000 et 100 000 environ.

Parmi ces polymères fixants, on peut citer les polyvinylpyrrolidones telles que celles commercialisées sous la dénomination Luviskol® K30 par la société BASF ; les polyvinylcaprolactames tels que ceux commercialisés sous la dénomination Luviskol® PLUS par la société BASF ; les copolymères poly(vinylpyrrolidone/acétate de vinyle) tels que ceux commercialisés sous la dénomination PVPVA® S630L par la société ISP, Luviskol® VA 73, VA 64, VA 55, VA 37 et VA 28 par la société BASF ; et les terpolymères poly(vinylpyrrolidone/acétate de vinyle/propionate de vinyle) tels que, par exemple, ceux commercialisés sous la dénomination Luviskol® VAP 343 par la société BASF.

Les polymères filmogènes cationiques utilisables selon la présente invention sont de préférence choisis parmi les polymères comportant des groupements amine primaire, secondaire, tertiaire et/ou quaternaire faisant partie de la chaîne polymère ou directement reliés à celle-ci, et ayant un poids moléculaire compris entre 500 et environ 5 000 000 et de préférence entre 1 000 et 3 000 000.

Parmi ces polymères, on peut citer plus particulièrement les polymères cationiques suivants:
(1) les homopolymères ou copolymères dérivés d'esters ou d'amides acryliques ou méthacryliques et comportant au moins un des motifs de formules suivantes: 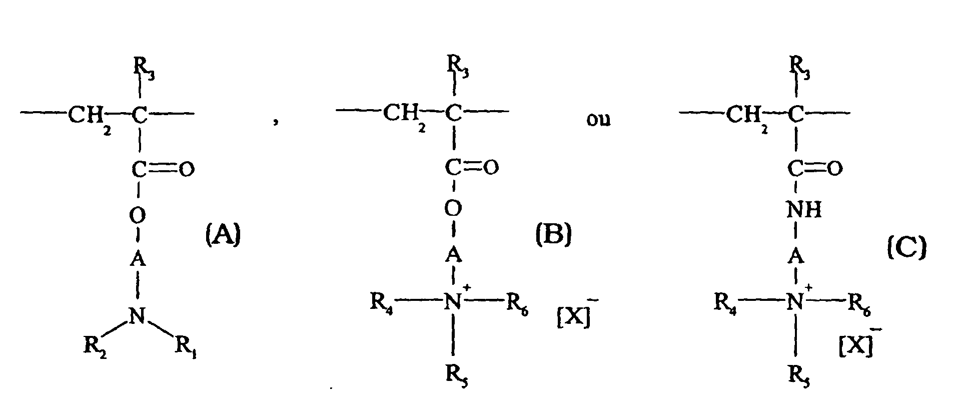 dans lesquelles:
   R₃ désigne un atome d'hydrogène ou un radical CH₃;
   A est un groupe alkyle linéaire ou ramifié de 1 à 6 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle;
   R₁ et R₂ représentent hydrogène ou un groupe alkyle ayant de 1 à 6 atomes de carbone;
   X désigne un anion méthosulfate ou un halogénure tel que chlorure ou bromure.

   Les copolymères de la famille (1) contiennent en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs, des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer :- les copolymères d'acrylamide et de diméthylaminoéthyl méthacrylate quaternisé au sulfate de diméthyle ou avec un halogénure de diméthyle tels que celui vendu sous la dénomination HERCOFLOC par la société HERCULES,
   - les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone/acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthylaminoéthyle/vinylcaprolactame/vinylpyrrolidone tel que le produit vendu sous la dénomination GAFFIX VC 713 par la société ISP,
   - et le copolymère vinylpyrrolidone/méthacrylamide de diméthylaminopropyle quaternisé tel que le produit vendu sous la dénomination "GAFQUAT HS 100" par la société ISP.
(2) les polysaccharides quaternisés décrits plus particulièrement dans les brevets américains 3.589.578 et 4.031.307 tel que les gommes de guar contenant des groupements cationiques trialkylammonium.
   De tels produits sont commercialisés notamment sous les dénominations commerciales de JAGUAR C13 S, JAGUAR C 15, JAGUAR C 17 par la société MEYHALL.
(3) les copolymères quaternaires de vinylpyrrolidone et de vinylimidazole tels que les produits commercialisés par BASF sous l'appellation Luviquat TFC;
(4) les chitosanes ou leurs sels;
   les sels utilisables sont en particulier les acétate, lactate, glutamate, gluconate ou le pyrrolidone carboxylate de chitosane .
   Parmi ces composés, on peut citer le chitosane ayant un taux de désacétylation de 90,5% en poids vendu sous la dénomination KYTAN BRUT STANDARD par la société ABER TECHNOLOGIES, le pyrrolidone carboxylate de chitosane vendu sous la dénomination KYTAMER PC par la société AMERCHOL.
(5) les dérivés de cellulose cationiques tels que les copolymères de cellulose ou de dérivés de cellulose greffés avec un monomère hydrosoluble comportant un ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyloxyéthyl triméthylammonium, de méthacrylamidopropyl triméthylammonium, de diméthyl-diallylammonium.

Les produits commercialisés répondant à cette définition sont plus particulièrement les produits vendus sous la dénomination "CELQUAT L 200" et "CELQUAT H 100" par la Société National Starch.

Dans la présente invention, on peut aussi utiliser à titre de polymère fixant des polymères hydrocarbonés à greffons silicones et des silicones à greffons hydrocarbonés. On peut aussi utiliser des polyuréthanes. Ces différents composés peuvent être non ioniques, cationiques, anioniques ou amphotères.

Un polyuréthane fixant utilisé dans la présente invention peut avantageusement comprendre en outre au moins une séquence polysiloxane et son motif répétitif de base répond par exemple à la formule générale (VII) :

-O-P-O-CO-NH-R-NH-CO- (VII)

dans laquelle :
- P est un segment polysiloxanique, et
- R est un groupe divalent choisi parmi les groupes alkylènes de type aromatique, aliphatique en C₁ à C₂₀, cycloaliphatique en C₁ à C₂₀, ces groupes étant substitués ou non.

Avantageusement, le segment polysiloxanique P répond à la formule générale (VIII) ci-après : 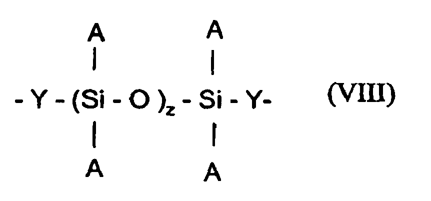 dans laquelle:
- les groupes A, qui peuvent être identiques ou différents, sont choisis parmi d'une part les groupes hydrocarbonés monovalents en C₁ à C₂₀ exempts ou substantiellement exempts d'insaturation éthylénique et, d'autre part, les groupes aromatiques,
- Y représente un groupe hydrocarboné divalent, et
- z représente un nombre entier, choisi de telle sorte que la masse moléculaire moyenne du segment polysiloxane soit comprise entre 300 et 10 000.

En général, le groupe divalent Y est choisi parmi les groupes alkylènes de formule -(CH₂)ₐ-, dans laquelle a représente un nombre entier pouvant être compris entre 1 et 10.

Les groupes A peuvent être choisis parmi les groupes alkyles, en particulier les groupes méthyle, éthyle, propyle, isopropyle, butyle, pentyle, hexyle, octyle, décyle, dodécyle et octadécyle ; les groupes cycloalkyle, en particulier le groupe cyclohexyle ; les groupes aryle, notamment phényle et naphtyle ; les groupes arylalkyle, notamment benzyle et phényléthyle, ainsi que les groupes tolyle et xylyle.

A titre d'exemples de polyuréthane fixant, on peut notamment citer le copolymère acide diméthylolpropionique/isophorone-diisocyanate/néopentylglycol/polyesterdiols (connu aussi sous le nom de polyuréthane-1, appellation INCI) vendu sous la marque Luviset® PUR par la société BASF, le copolymère acide diméthylolpropionique/isophorone-diisocyanate/néopentylglycol/polyesterdiols/diamine siliconée (connu aussi sous le nom de polyuréthane-6, appellation INCI) vendu sous la marque Luviset® Si PUR A par la société BASF.

La composition capillaire de coiffage selon l'invention comprend de préférence les polymères fixants en une quantité comprise entre 0,1 et 20 % en poids, de préférence entre 0,5% et 15% en poids par rapport au poids total de la composition selon l'invention.

Le propulseur utilisé dans la présente invention est le diméthyléther dans les proportions de 20 à 60% du poids total de la composition.

Le milieu cosmétiquement acceptable comprend de l'eau et éventuellement un solvant cosmétiquement acceptable. Ce solvant cosmétiquement acceptable est notamment choisi parmi les alcools inférieurs en C₁-C₄ comme l'éthanol, l'isopropanol, le tertio-butanol, le n-butanol ; les polyols comme le propylèneglycol, et les éthers de polyols ; l'acétone ; et leurs mélanges, le solvant particulièrement préféré étant l'éthanol.

La proportion en eau peut être comprise entre 20 et 95% en poids par rapport au poids total des compositions. De manière avantageuse, le milieu est un mélange hydroalcoolique. La proportion en alcool du mélange est comprise entre 0 et 70% en poids, de préférence entre 0 et 55% en poids et encore plus préférentiellement entre 10 et 55 % en poids par rapport au poids total des compositions.

La composition capillaire fixante selon l'invention peut contenir en outre au moins un adjuvant choisi parmi les silicones sous forme soluble, dispersée, micro-dispersée, les agents tensio-actifs non-ioniques, anioniques, cationiques et amphotères, les céramides et pseudo-céramides, les vitamines et pro-vitamines dont le panthénol, les huiles végétales, animales, minérales et synthétiques, les cires autres que les céramides et pseudo-céramides, les filtres solaires hydrosolubles et liposolubles, siliconés ou non siliconés, les pigments minéraux et organiques, colorés ou non colorés, les colorants, les agents nacrants et opacifiants, les agents séquestrants, les agents plastifiants, les agents solubilisants, les agents acidifiants, des agents alcalinisants, les agents épaississants minéraux et organiques, les agents anti-oxydants, les hydroxyacides, les agents de pénétration, les parfums et les agents conservateurs.

L'homme de métier veillera à choisir les éventuels additifs et leur quantité de manière à ce qu'ils ne nuisent pas aux propriétés des compositions de la présente invention.

Ces additifs sont présents dans la composition selon l'invention en une quantité allant de 0 à 20 % en poids par rapport au poids total de la composition.

Les compositions conformes à l'invention peuvent être utilisées en tant que compositions de fixation et/ou de maintien des cheveux, compositions de soin de cheveux, shampooings, compositions de conditionnement des cheveux, telles que des compositions destinées à apporter de la douceur aux cheveux, ou encore des compositions de maquillage des cheveux.

L'invention concerne aussi un dispositif aérosol à deux compartiments comprenant contenant:
a)une composition capillaire de coiffage qui comprend dans un milieu aqueux cosmétiquement acceptable au moins un copolymère à base de motifs N-vinyl pyrrolidone et N-vinyl imidazole ayant un poids moléculaire supérieur à 10 Kdaltons, et
b) un gaz comprimé choisi parmi l'air, l'azote, le gaz carbonique, et leurs mélanges.

Avantageusement, dans le dispositif à deux compartiments, la composition capillaire de coiffage contient en outre un polymère fixant différent du copolymère à base de motifs N-vinyl pyrrolidone et de N-vinyl imidazole.

Plus avantageusement, dans le dispositif à deux compartiments, la composition comprend par rapport au poids total de la composition:
- 2 % en poids d'un copolymère à base de motifs de N-vinyl pyrrolidone et de N-vinyl imidazole ayant un poids moléculaire (ou PM) supérieur à 10 KDaltons, et du diméthyléther comme agent propulseur, et
- 1 % en poids d'un polyuréthane fixant.

La présente invention concerne également l'utilisation du dispositif pour appliquer une laque sur les cheveux par vaporisation de son contenu.

La présente invention concerne aussi l'utilisation du produit vaporisé par le dispositif aérosol selon l'invention comme laque pour cheveux.

La présente invention concerne également un procédé de traitement, caractérisé en ce qu'on vaporise la composition capillaire de coiffage contenue dans le dispositif aérosol selon l'une quelconque des revendications précédentes, sur les cheveux mouillés ou non.

Les exemples suivants illustrent la présente invention et ne doivent être considérés en aucune manière comme limitant l'invention.

### EXEMPLE 1

La demanderesse a réalisé une composition conforme à l'invention formulée comme suit :

| | |
|---|---|
| Luvitec VPI 55 K 61 | 4% |
| Eau | 30% |
| Ethanol | 31% |
| DME | 35% |

### EXEMPLE 2

La demanderesse a réalisé une composition conforme à l'invention formulée comme suit :

| | | |
|---|---|---|
| Luvitec VPI 55 K 61 | (MA) | 1% |
| Polymère classique (Luviset® PUR) | (MA) | 4% |
| Eau | | 40% |
| Ethanol | | 20% |
| DME | | 35% |
| | | MA : matière active |

Les compositions des exemples 1 et 2 conditionnées dans un dispositif aérosol et pulvérisées sur les cheveux confèrent à ceux-ci un très bon maintien associé à un très bon niveau cosmétique.

### EXEMPLE 3

On réalise une compositions de coiffage comprenant:
Copolymère N-vinylpyrrolidone / N-vinylimidazole à 1% MA (1)
Polymère acide diméthylol proponique / diisocyanate / néopentylglycol / polyester diols à 1% MA (2)

(1) LUVITEC VPI K 65
(2) LUVISET Si PUr

La formulation est la suivante:

| | | |
|---|---|---|
| (1) | (MA) | 2 % |
| (2) | (MA) | 1 % |
| Eau | | 40%? |
| Ethanol | | 20% |
| DME | | 35% |

On a introduit la composition préparée ci-dessus dans un dispositif de distribution aérosol commercialisé sous le nom EP SPRAY SYSTEM S. Une valve de référence 6001 format D6 est fixée sur un bidon aérosol classique et le diffuseur est un diffuseur à buse tourbillonnaire.

La poche est remplie avec la composition et de l'air comprimé est introduit entre la poche et le bidon.

Cette composition conditionnée dans un dispositif aérosol et pulvérisée sur les cheveux confère à ceux-ci un très bon maintien associé à un très bon niveau cosmétique.

## Revendications

1. Dispositif aérosol contenant une composition capillaire de coiffage qui comprend dans un milieu aqueux cosmétiquement acceptable au moins un copolymère à base de motifs N-vinyl pyrrolidone et N-vinyl imidazole ayant un poids moléculaire supérieur à 10 KDaltons, et du diméthyléther comme agent propulseur.

2. Dispositif aérosol selon la revendication 1, **caractérisé en ce que** le copolymère à base de motifs N-vinyl pyrrolidone et de N-vinyl imidazole a un poids moléculaire compris entre 100 et 5000 Kdaltons.

3. Dispositif selon la revendication 2, **caractérisé en ce que** le copolymère à base de motifs N-vinyl pyrrolidone et de N-vinyl imidazole a un poids moléculaire compris entre 500 et 1500 KDaltons.

4. Dispositif selon l'une quelconque des revendications 1 à 3, **caractérisé en ce que** le copolymère est formé par 10 à 90% en poids de motifs N-vinyl pyrrolidone et par 10 à 90% en poids de motifs N-vinyl imidazole.

5. Dispositif selon la revendication 4, **caractérisé en ce que** le copolymère est formé par 40 à 60% en poids de motifs N-vinyl pyrrolidone et par 60 à 40% en poids de motifs N-vinyl imidazole.

6. Dispositif selon lune quelconque des revendications 1 à 5, **caractérisé en ce que** la composition capillaire de coiffage contient de 0,1 à 15% en poids de copolymère à base de motifs N-vinyl pyrrolidone et de N-vinyl imidazole par rapport au poids total de la composition.

7. Dispositif selon l'une quelconque des revendications 1 à 6, **caractérisé par le fait que** la composition capillaire de coiffage contient en outre un polymère fixant différent du copolymère à base de motifs N-vinyl pyrrolidone et de N-vinyl imidazole.

8. Dispositif selon la revendication 7, **caractérisé en ce que** le polymère fixant additionnel est anionique, cationique, amphotère ou non-ionique.

9. Dispositif selon la revendication 7 ou 8, **caractérisé en ce que** le ou les polymères fixants additionnels sont choisis parmi les copolymères d'acide acrylique tels que le terpolymère acide crotonique/acrylate d'éthyle/N-tertiobutylacrylamide, le terpolymère acide crotonique/acétate de vinyle/terbutyl benzoate de vinyle, le terpolymère acide méthacrylique/acrylate d'éthyle/acrylate de terbutyle, les polymères siliconés greffés, à squelette polysiloxanique greffé par au moins un radical hydrocarboné, les polyuréthanes.

10. Dispositif selon l'une quelconque des revendications 7 à 9, **caractérisé en ce que** la composition contient de 0,1 à 20% en poids de polymère fixant additionnel par rapport au poids total de la composition, et de préférence 0,5 à 15%.

11. Dispositif selon l'une quelconque des revendications 1 à 10, **caractérisé en ce que** la composition contient de 20 à 60% de diméthyléther du poids total de la composition.

12. Dispositif selon l'une quelconque des revendications 1 à 11, **caractérisé en ce que** le milieu aqueux cosmétiquement acceptable comprend de l'eau seule ou en mélange avec un solvant cosmétiquement acceptable.

13. Dispositif selon la revendication 12, **caractérisé en ce que** le solvant est choisi parmi les alcools en C₁-C₄, les polyols, les éthers de polyols, l'acétone et leurs mélanges.

14. Dispositif selon la revendication 13, **caractérisé en ce que** le solvant est un alcool en C₁-C₄, tel que l'éthanol, l'isopropanol.

15. Dispositif selon l'une quelconque des revendications 12 à 14, **caractérisé en ce que** la composition contient de 20 à 95% d'eau en poids par rapport au poids total de la composition.

16. Dispositif selon l'une quelconque des revendications 13 à 15, **caractérisé en ce que** la composition contient de 0 à 70% en poids d'alcool en C₁-C₄, de préférence de 0 à 55% en poids et encore plus préférentiellement de 10 à 55 % en poids par rapport au poids total de la composition.

17. Dispositif selon l'une quelconque des revendications 1 à 16, **caractérisé par le fait que** la composition coiffante comprend un adjuvant choisi parmi les silicones sous forme soluble, dispersée, micro-dispersée, les agents tensio-actifs non-ioniques, anioniques, cationiques et amphotères, les céramides et pseudo-céramides, les vitamines et pro-vitamines dont le panthénol, les huiles végétales, animales, minérales et synthétiques, les cires autres que les céramides et pseudo-céramides, les filtres solaires hydrosolubles et liposolubles, siliconés ou non siliconés, les pigments minéraux et organiques, colorés ou non colorés, les colorants, les agents nacrants et opacifiants, les agents séquestrants, les agents plastifiants, les agents solubilisants, les agents acidifiants, des agents alcalinisants, les agents épaississants minéraux et organiques, les agents anti-oxydants, les hydroxyacides, les agents de pénétration, les parfums et les agents conservateurs.

18. Produit à deux composants, **caractérisé par le fait que** le premier composant est constitué par une composition comprenant dans un milieu aqueux cosmétiquement acceptable au moins un copolymère à base de motifs N-vinyl pyrrolidone et N-vinyl imidazole ayant un poids moléculaire supérieur à 10 Kdaltons et que le second composant comprend un agent propulseur constitué par le diméthyléther, l'ensemble étant conditionné dans un dispositif aérosol.

19. Dispositif aérosol à deux compartiments comprenant contenant:
a)une composition capillaire de coiffage qui comprend dans un milieu aqueux cosmétiquement acceptable au moins un copolymère à base de motifs N-vinyl pyrrolidone et N-vinyl imidazole ayant un poids moléculaire supérieur à 10 Kdaltons, et
b) un gaz comprimé choisi parmi l'air, l'azote, le gaz carbonique, et leurs mélanges.

20. Dispositif à deux compartiments selon la revendication 19, **caractérisé par le fait que** la composition capillaire de coiffage contient en outre un polymère fixant différent du copolymère à base de motifs N-vinyl pyrrolidone et de N-vinyl imidazole.

21. Utilisation du produit vaporisé par le dispositif aérosol selon 19, 20, l'une quelconque des revendications 1 à 17 et 19,20, comme laque pour cheveux.

22. Utilisation du dispositif selon l'une quelconque des revendications 19;20, 1 à 17 et pour appliquer une laque sur les cheveux par vaporisation de son contenu.

23. Procédé de traitement capillaire, **caractérisé en ce qu'**on vaporise la composition capillaire de coiffage contenue dans le dispositif aérosol selon l'une quelconque des revendications 1 à 17 et 19; 20, sur les cheveux mouillés ou non.

## Patentansprüche

1. Aerosolvorrichtung, die eine Zusammensetzung für die Frisurengestaltung der Haare enthält, die in einem kosmetisch akzeptablen wässrigen Medium mindestens ein Copolymer auf der Basis von N-Vinylpyrrolidoneinheiten und N-Vinylimidazoleinheiten mit einem Molekulargewicht über 10 kDalton und als Treibmittel Dimethylether enthält.

2. Aerosolvorrichtung nach Anspruch 1, **dadurch gekennzeichnet, dass** das Copolymer auf der Basis von N-Vinylpyrrolidoneinheiten und N-Vinylimidazoleinheiten ein Molekulargewicht von 100 bis 5 000 kDalton aufweist,

3. Vorrichtung nach Anspruch 2, **dadurch gekennzeichnet, dass** das Copolymer auf der Basis von N-Vinylpyrrolidoneinheiten und N-Vinylimidazoleinheiten ein Molekulargewicht von 500 bis 1 500 kD aufweist.

4. Vorrichtung nach einem der Ansprüche 1 bis 3, **dadurch gekennzeichnet, dass** das Copolymer aus 10 bis 90 Gew.-% N-Vinylpyrrolidoneinheiten und 10 bis 90 Gew.-% N-Vinylimidazoleinheiten gebildet ist.

5. Vorrichtung nach Anspruch 4, **dadurch gekennzeichnet, dass** das Copolymer aus 40 bis 60 Gew.-% N-Vinylpyrrolidoneinheiten und 60 bis 40 Gew.-% N-Vinylimidazoleinheiten gebildet ist.

6. Vorrichtung nach einem der Ansprüche 1 bis 5, **dadurch gekennzeichnet, dass** die Zusammensetzung für die Frisurengestaltung der Haare 0, 1 bis 15 Gew,-% Copolymer auf der Basis von N-Vinylpyrrolidoneinheiten und N-Vinylimidazoleinheiten, bezogen auf das Gesamtgewicht der Zusammensetzung, enthält.

7. Vorrichtung nach einem der Ansprüche 1 bis 6, **dadurch gekennzeichnet, dass** die Zusammensetzung für die Frisurengestaltung der Haare ferner ein festigendes Polymer enthält, das von dem Copolymer auf der Basis von N-Vinylpyrrolidoneinheiten und N-Vinylimidazoleinheiten verschieden ist.

8. Vorrichtung nach Anspruch 7, **dadurch gekennzeichnet, dass** das zusätzliche festigende Polymer anionisch, kationisch, amphoter oder nichtionisch ist.

9. Vorrichtung nach dem Anspruch 7 oder 8, **dadurch gekennzeichnet, dass** das oder die zusätzliche(n) festigende(n) Polymer(e) unter den Copolymeren von Acrylsäure, wie dem Crotonsäure/Ethylacryl/N-t-Butylacrylamid-Terpolymer, dem Crotonsäure/Vinylacetat/Vinyl-t-butylbenzoat-Terpolymer, dem Methacrylsäure/ Ethylacrylat/ t-Butylacrylat-Terpolymer, gepfropften Siliconpolymeren mit Polysiloxangerüst, auf das mindestens eine Kohlenwasserstoffgruppe gepfropft ist, und Polyurethanen ausgewählt sind.

10. Vorrichtung nach einem der Ansprüche 7 bis 9, **dadurch gekennzeichnet, dass** die Zusammensetzung 0,1 bis 20 Gew.-% zusätzliches festigendes Polymer, bezogen auf das Gesamtgewicht der Zusammensetzung, und vorzugsweise 0,5 bis 15 % enthält.

11. Vorrichtung nach einem der Ansprüche 1 bis 10, **dadurch gekennzeichnet, dass** die Zusammensetzung 20 bis 60 % Dimethylether, bezogen auf das Gesamtgewicht der Zusammensetzung, enthält.

12. Vorrichtung nach einem, der Ansprüche 1 bis 11, **dadurch gekennzeichnet, dass** das kosmetisch akzeptable wässrige Medium Wasser alleine oder Wasser im Gemisch mit einem kosmetisch akzeptablen Lösungsmittel enthält.

13. Vorrichtung nach Anspruch 12, **dadurch gekennzeichnet, dass** das Lösungsmittel unter den C₁₋₄-Alkoholen, Polyolen, Polyolethern, Aceton und deren Gemischen ausgewählt ist.

14. Vorrichtung nach Anspruch 13, **dadurch gekennzeichnet, dass** das Lösungsmittel ein C₁₋₄-Alkohol ist, wie Ethanol oder Isopropanol.

15. Vorrichtung nach einem der Ansprüche 12 bis 14, **dadurch gekennzeichnet, dass** die Zusammensetzung 20 bis 95 Gew.-% Wasser, bezogen auf das Gesamtgewicht der Zusammensetzung, enthält.

16. Vorrichtung nach einem der Ansprüche 13 bis 15, **dadurch gekennzeichnet, dass** die Zusammensetzung 0 bis 70 Gew.-% C₁₋₄₋Alkohol, vorzugsweise 0 bis 55 Gew.-% und noch bevorzugter 10 bis 55 Gew.-%, bezogen auf das Gesamtgewicht der Zusammensetzung, enthält.

17. Vorrichtung nach einem der Ansprüche 1 bis 16, **dadurch gekennzeichnet, dass** die Zusammensetzung für die Frisurengestaltung einen Zusatzstoff enthält, der unter den Siliconen in löslicher, dispergierter oder mikrodispergierter Form, nichtionischen, anionischen, kationischen und amphoteren grenzflächenaktiven Stoffen, Ceramiden und Pseudoceramiden, Vitaminen und Provitaminen, darunter Panthenol, pflanzlichen Ölen, tierischen Ölen, Mineralölen und synthetischen Ölen, Wachsen, die von den Ceramiden und Pseudoceramiden verschieden sind, wasserlöslichen und fettlöslichen, siliconierten oder nicht siliconierten Sonnenschutzfiltern, anorganischen und organischen, farbigen oder nicht farbigen Pigmenten, Farbstoffen, Perlglanzpigmenten und Trübungsmitteln, Maskierungsmitteln, Weichmachern, Lösungsvermittlern, Ansäuerungsmitteln, Alkalisierungsmitteln, anorganischen und organischen Verdickungsmitteln, Antioxidantien, Hydroxysäuren, Penetrationsmitteln, Parfums und Konservierungsmitteln ausgewählt ist.

18. Produkt mit zwei Komponenten, **dadurch gekennzeichnet, dass** die erste Komponente aus einer Zusammensetzung besteht, die in einem kosmetisch akzeptablen wässrigen Medium mindestens ein Copolymer auf der Basis von N-Vinylpyrrolidoneinheiten und N-Vinylimidazoleinheiten mit einer Molmasse über 10 kDalton enthält, und **dadurch**, dass die zweite Komponente ein Treibmittel umfasst, das aus Dimethylether besteht, wobei das Ganze in einer Aerosolvorrichtung konfektioniert ist.

19. Aerosolvorrichtung mit zwei Abteilungen, die enthalten:
a) eine Zusammensetzung zur Frisurengestaltung der Haare, die in einem kosmetisch akzeptablen wässrigen Medium mindestens ein Copolymer auf der Basis von N-Vinylpyrrolidoneinheiten und N-Vinylimidazoleinheiten mit einer Molmasse über 10 kDalton enthält, und
b) ein Treibgas, das unter Luft, Stickstoff, Kohlendioxid und deren Gemischen ausgewählt ist.

20. Vorrichtung mit zwei Abteilungen nach Anspruch 19, **dadurch gekennzeichnet, dass** die Zusammensetzung für die Frisurengestaltung der Haare ferner ein festigendes Polymer enthält, das von dem Copolymer auf der Basis von N-Vinylpyrrolidoneinheiten und N-Vinylimidazoleinheiten verschieden ist.

21. Verwendung des aus der Aerosolvorrichtung nach einem der Ansprüche 1 bis 17 und 19, 20 zerstäubten Produkts als Haarlack.

22. Verwendung der Vorrichtung nach einem der Ansprüche 1 bis 17 und 19, 20 zum Aufbringen eines Lacks auf die Haare durch Zerstäuben ihres Inhalts.

23. Verfahren zur Haarbehandlung, **dadurch gekennzeichnet, dass** die Zusammensetzung für die Frisurengestaltung der Haare, die in der Aerosolvorrichtung nach einem der Ansprüche 1 bis 17 und 19, 20 enthalten ist, auf die feuchten oder nicht feuchten Haaren gesprüht wird.

## Claims

1. Aerosol device containing a hair composition for hair styling which comprises, in a cosmetically acceptable aqueous medium, at least one copolymer based on N-vinylpyrrolidone and N-vinylimidazole units having a molecular weight greater than 10 KDaltons, and dimethyl ether as propelling agent.

2. Aerosol device according to Claim 1, **characterized in that** the copolymer based on N-vinylpyrrolidone and N-vinylimidazole units has a molecular weight of between 100 and 5000 KDaltons.

3. Device according to Claim 2, **characterized in that** the copolymer based on N-vinylpyrrolidone and N-vinylimidazole units has a molecular weight of between 500 and 1500 KDaltons.

4. Device according to any one of Claims 1 to 3, **characterized in that** the copolymer consists of 10 to 90% by weight of N-vinylpyrrolidone units and of 10 to 90% by weight of N-vinylimidazole units.

5. Device according to Claim 4, **characterized in that** the copolymer consists of 40 to 60% by weight of N-vinylpyrrolidone units and of 60 to 40% by weight of N-vinylimidazole units.

6. Device according to any one of Claims 1 to 5, **characterized in that** the hair composition for hair styling contains from 0.1 to 15% by weight of copolymer based on N-vinylpyrrolidone and N-vinylimidazole units relative to the total weight of the composition.

7. Device according to any one of Claims 1 to 6, **characterized in that** the hair composition for hair styling additionally contains a fixing polymer different from the copolymer based on N-vinylpyrrolidone and N-vinylimidazole units.

8. Device according to Claim 7, **characterized in that** the additional fixing polymer is anionic, cationic, amphoteric or nonionic.

9. Device according to Claim 7 or 8, **characterized in that** the additional fixing polymers are chosen from acrylic acid copolymers such as the terpolymer crotonic acid/ethyl acrylate/N-tert-butylacrylamide, the terpolymer crotonic acid/vinyl acetate/vinyl tert-butylbenzoate, the terpolymer methacrylic acid/ethyl acrylate/tert-butyl acrylate, the graft silicone polymers, containing a polysiloxane backbone grafted with at least one hydrocarbon radical, polyurethanes.

10. Device according to any one of Claims 7 to 9, **characterized in that** the composition contains from 0.1 to 20% by weight of additional fixing polymer relative to the total weight of the composition, and preferably 0.5 to 15%.

11. Device according to any one of Claims 1 to 10, **characterized in that** the composition contains from 20 to 60% of dimethyl ether of the total weight of the composition.

12. Device according to any one of Claims 1 to 11, **characterized in that** the cosmetically acceptable aqueous medium comprises water alone or as a mixture with a cosmetically acceptable solvent.

13. Device according to Claim 12, **characterized in that** the solvent is chosen from C₁-C₄ alcohols, polyols, polyol ethers, acetone and mixtures thereof.

14. Device according to Claim 13, **characterized in that** the solvent is a C₁-C₄ alcohol, such as ethanol, isopropanol.

15. Device according to any one of Claims 12 to 14, **characterized in that** the composition contains from 20 to 95% of water by weight relative to the total weight of the composition.

16. Device according to any one of Claims 13 to 15, **characterized in that** the composition contains from 0 to 70% by weight of C₁-C₄ alcohol, preferably from 0 to 55% by weight and still more preferably from 10 to 55% by weight relative to the total weight of the composition.

17. Device according to any one of Claims 1 to 16, **characterized in that** the hairstyling composition comprises an adjuvant chosen from silicones in soluble, dispersed or microdispersed form, nonionic, anionic, cationic and amphoteric surfactants, ceramides and pseudoceramides, vitamins and provitamins including panthenol, vegetable, animal, mineral and synthetic oils, waxes other than ceramides and pseudoceramides, silicone or nonsilicone, water-soluble and fat-soluble sunscreens, coloured or noncoloured, inorganic and organic pigments, colorants, pearlescent and opacifying agents, sequestering agents, plasticizing agents, solubilizing agents, acidifying agents, alkalinizing agents, inorganic and organic thickening agents, antioxidants, hydroxy acids, penetrating agents, perfumes and preservatives.

18. Product containing two components, **characterized in that** the first component consists of a composition comprising, in a cosmetically acceptable aqueous medium, at least one copolymer based on N-vinylpyrrolidone and N-vinylimidazole units having a molecular weight greater than 10 KDaltons and **in that** the second component comprises a propelling agent consisting of dimethyl ether, the whole being packaged in an aerosol device.

19. Two-compartment aerosol device comprising:
a) a hair composition for hair styling which comprises, in a cosmetically acceptable aqueous medium, at least one copolymer based on N-vinylpyrrolidone and N-vinylimidazole units having a molecular weight greater than 10 KDaltons, and
b) a compressed gas chosen from air, nitrogen, carbon dioxide, and mixtures thereof.

20. Two-compartment device according to Claim 19, **characterized in that** the hair composition for hair styling additionally contains a fixing polymer different from the copolymer based on N-vinylpyrrolidone and N-vinylimidazole units.

21. Use of the product sprayed by the aerosol device according to any one of Claims 1 to 17 and 19, 20, as a hair lacquer.

22. Use of the device according to any one of Claims 1 to 17 and 19, 20, for applying a lacquer to the hair by spraying its contents.

23. Method of hair treatment, **characterized in that** the hair composition for hair styling contained in the aerosol device according to any one of Claims 1 to 17 and 19, 20 is sprayed onto the hair which is wet or not.
